# FASCICULE DE BREVET EUROPEEN

(11) **EP 0 765 667 B1**
(45) Date de publication et mention de la délivrance du brevet: **03.01.2001**
(21) Numéro de dépôt: 96401785.9
(22) Date de dépôt: 13.08.1996
(51) Int. Cl.: A61K 35/74, A61K 7/48

(54) **Fraction ribosomale et composition la contenant**
Ribosomenfraktion und Zusammensetzung, die sie enthält
Ribosomal fraction and composition containing it

(30) Priorité: 28.09.1995 FR 9511404
(43) Date de publication de la demande: 02.04.1997
(73) Titulaire: L'OREAL, 75008 Paris (FR)
(72) Inventeur: Pineau, Nathalie, Rés. des jardins du Clain, 86000 Poitiers (FR); Breton, Lionel, 78000 Versailles (FR); Martin, Richard, 37210 Rochecorbon (FR)
(74) Mandataire: Andral, Christophe André Louis

(56) Documents cités:
- EP-A- 0 238 407
- EP-A- 0 657 530
- FR-A- 2 693 654
- FR-A- 2 700 172
- CANCER RESEARCH, vol. 40, Mai 1980, pages 1501-1505, XP002004701 URBAN ET AL: "TUMOR-IMMUNOTHERAPEUTIC EFFICACY OF SERRATIA MARCESCENS POLYRIBOSOMES"

## Description

La présente invention concerne une fraction ribosomale d'au moins une bactérie filamenteuse non photosynthétique.

Les ribosomes sont des formations corpusculaires de 140 à 230 Angströms constituées d'acides ribonucléiques associés à des protéines et qui sont présents dans les cellules de tous les organismes, en particulier les bactéries. Les ribosomes sont le siège des réactions complexes qui permettent la synthèse des protéines grâce au complexe multienzymatique qui les constitue.

Chez les procaryotes, les ribosomes ont une constante de sédimentation de l'ordre de 70S (Svedberg : Unité internationale mesurant la vitesse de sédimentation de corps soumis à une centrifugation standard).

Morphologiquement, les ribosomes sont divisés en 2 sous-unités de tailles inégales par un sillon perpendiculaire à leur grand axe. Ces 2 sous-unités ont respectivement une constante de sédimentation de l'ordre de 50 S et 30 S. La sous-unité 50 S contient deux acides ribonucléiques respectivement de 23 S et 5 S et une trentaine de protéines, alors que la sous-unité 30 S contient un acide ribonucléique de 16 S et une vingtaine de protéines.

On comprend donc que l'expression "fraction ribosomale" correspond à la fraction de milieu enrichie en ribosomes que l'on peut obtenir, par exemple après centrifugation, en séparant les différents organites constituant les cellules d'un organisme.

Ainsi, une fraction ribosomale peut donc contenir, quelque soit son degré de pureté, soit au moins un ribosome entier, soit au moins un élément constitutif d'un ribosome ou soit un élément constitutif de l'une des sous-unités d'un ribosome.

A ce jour, des fractions ribosomales sont utilisées par exemple en cosmétique dans la préparation de compositions destinées à retarder le vieillissement de la peau par stimulation de la croissance cellulaire et modulation de la maturation du tissu conjonctif (EP-A-631773). En médecine, on connaît des compositions destinées à renforcer les défenses immunitaires en particulier de patients ayant subi de graves brûlures et sensibles de ce fait aux infections opportunistes causées par des bactéries, des virus ou des champignons (WO 91/11174) ou encore en ce qui concerne les maladies de la sphère otorhinolaryngologique (FR 2253499, FR 2360314, FR 2388563, FR 2674755, ZA 8801071), les allergies (US 4946945). Les fractions ribosomales sont également utilisées dans la préparation d'adjuvants pour vaccins (FR 2374911, DE 1617809).
Généralement, les fractions ribosomales utilisées dans ces inventions sont préparées à partir de bactéries Gram négatives comme par exemple celles de la famille des Enterobacteriacées (e. g. *Klebsiella pneumoniae, Escherichia coli, Serratia marcessens),* ou de la famille des Pasteurellacées (e. g. *Haemophilus influenzae),* ou de bactéries Gram positives comme par exemple celles de la famille des Bacillus (e. g. *Bacilllus subtilis),* de la famille des Streptococcacées (e. g. *Streptococcus pneumoniae, Streptococcus pyogenes* Gr. A), ou de la famille des Lactobacillacées (e. g. *Acidophilus, Bifidum),* ou à partir de levures comme par exemple *Candida albicans* ou *Candida tropicalis.*

Bien que les fractions ribosomales de l'art antérieur donnent satisfaction, il n'en demeure pas moins qu'il reste intéressant de rechercher de nouvelles fractions ribosomales préparées à partir d'organismes jusqu'alors inutilisés dans ce domaine.

Un des buts de l'invention est donc de proposer une nouvelle fraction ribosomale susceptible d'être utilisée au moins dans les domaines indiqués ci-dessus, éventuellement dans de nouveaux domaines.

L'invention a donc pour objet, à titre de produit nouveau, une fraction ribosomale préparée à partir d'au moins une bactérie filamenteuse non photosynthétique.

La fraction ribosomale, comme il a été précisé précédement, peut contenir quelque soit son degré de pureté, soit au moins un ribosome entier, soit au moins un élément constitutif d'un ribosome ou soit un élément constitutif de l'une des sous-unités d'un ribosome.

Le système immunitaire comprend un ensemble de cellules spécialisées soumises à de multiples mécanismes de contrôle assurant leur renouvellement, leur activation et leur différenciation, indispensables à un niveau normal d'immunocompétence. Le rôle du système immunitaire est de discriminer le soi du non soi pour éliminer les agents pathogènes et les tumeurs spontanées. Toute déplétion cellulaire, toute dysrégulation immunitaire ou tout déficit fonctionnel est susceptible de favoriser la survenue de manifestations pathologiques caractérisées par la perturbation des mécanismes de reconnaissance du soi vis-à-vis du non soi, et une plus grande sensibilité vis-à-vis des agressions microbiennes et des processus néoplasiques.

La peau constitue l'organe le plus important de l'organisme et est reconnue comme l'un des principaux éléments actifs du système de défense immunitaire. Trois types de cellules épidermiques participent à ce système : les kératinocytes, les mélanocytes et les cellules de Langerhans. Ces cellules que l'on ne retrouve qu'au niveau de la peau, jouent un rôle primordial dans la réponse immunitaire et en particulier dans la présentation antigénique.

La peau saine est capable de se défendre des agressions extérieures grâce aux moyens mis à sa disposition. Néanmoins, elle est soumise à l'agression permanente de l'environnement, de produits chimiques, de radiations. En particulier les cellules de Langerhans sont la cible privilégiée des rayonnements ultraviolets.

Ces agressions se traduisent par un effet suppresseur des défenses immunitaires entraînant une baisse des résistances aux agents pathogènes et une augmentation de l'incidence de certains cancers.

Pour aider la peau à remplir sa fonction immunitaire, des produits de stimulation du système immunitaire cutanée sont d'un grand intérêt.

On sait d'autre part que le système immunitaire et plus particulièrement celui de la peau s'affaiblit au cours du vieillissement chronobiologique.
Cet affaiblissement survient également au cours du vieillissement photo-induit. Un effet immunostimulateur peut alors rétablir les fonctions immunitaires et plus particulièrement celles de l'épiderme en renforçant les défenses naturelles de la peau.

Un autre but de l'invention est donc de proposer un nouvel immunostimulant, plus particulièrement du système immunitaire de la peau.

Un autre objet de l'invention concerne une composition cosmétique ou pharmaceutique, caractérisée en ce qu'elle comprend au moins une fraction ribosomale d'au moins une bactérie filamenteuse non photosynthétique.

La fraction ribosomale d'au moins une bactérie filamenteuse non photosynthétique possède un remarquable pouvoir de stimulation du système immunitaire.

Ainsi, l'invention a plus précisément pour objet une composition cosmétique ou pharmaceutique comprenant, à titre de principe immunostimulant, au moins une fraction ribosomale d'au moins une bactérie filamenteuse non photosynthétique.

Préférentiellement, la fraction ribosomale est destinée à stimuler le système immunitaire de la peau. Cette stimulation du système immunitaire de la peau est particulièrement intéressante au cours du vieillissement chronobiologique et/ou du photovieillissement.

La fraction ribosomale de l'invention provient de bactéries choisies parmi les bactéries filamenteuses non photosynthétiques telles que définies selon la classification du Bergey's Manual of Systematic Bacteriology (vol. 3, sections 22 et 23, 9°édition, 1989), parmi lesquelles on peut citer les bactéries appartenant à l'ordre des Beggiatoales, et plus particulièrement les bactéries appartenant aux genres *Beggiatoa, Vitreoscilla, Flexithrix* ou *Leucothrix.*

Les bactéries qui viennent d'être définies et dont plusieurs ont déjà été décrites ont généralement un habitat aquatique et peuvent être trouvées notamment dans des eaux marines ou dans des eaux thermales. Parmi les bactéries utilisables, on peut citer par exemple :
*Vitreoscilla filiformis* (ATCC 15551)
*Vitreoscilla beggiatoïdes* (ATCC 43181)
*Beggiatoa alba* (ATCC 33555)
*Flexithrix dorotheae* (ATCC 23163)
*Leucothrix mucor* (ATCC 25107)
*Sphaerotilus natans* (ATCC 13338)

Préférentiellement, on utilise selon l'invention une souche de *Vitreoscilla filiformis.*

Toute méthode de préparation de fraction ribosomale connue de l'homme du métier peut être utilisée selon l'invention.

On peut en particulier citer les méthodes décrites par Norris et Ribbons dans "Methods in Microbiology", 1973, (Academic Press).

Dans les compositions selon l'invention, la fraction ribosomale d'au moins une bactérie filamenteuse non photosynthétique représente de 0,0001 % à 20 % du poids total de la composition et préférentiellement de 0,01 à 10 % du poids total de la composition.

Selon que la fraction est utilisée dans une composition qui doit être ingérée, injectée ou appliquée sur la peau (sur toute zone de la peau du corps), les cheveux, les ongles ou les muqueuses (buccale, jugale, gingivale, génitale, anale, conjonctive), cette composition peut se présenter sous toutes les formes galéniques normalement utilisées.

Pour une application topique sur la peau, la composition peut avoir la forme notamment de solution aqueuse ou huileuse ou de dispersion du type lotion ou sérum, d'émulsions de consistance liquide ou semi-liquide du type lait, obtenues par dispersion d'une phase grasse dans une phase aqueuse (H/E) ou inversement (E/H), ou de suspensions ou émulsions de consistance molle du type crème ou gel aqueux ou anhydres, ou encore de microcapsules ou microparticules, ou de dispersions vésiculaires de type ionique et/ou non ionique. Ces compositions sont préparées selon les méthodes usuelles.

Elles peuvent être également utilisées pour les cheveux ou le cuir chevelu sous forme de solutions aqueuses, alcooliques ou hydroalcooliques, ou sous forme de crèmes, de gels, d'émulsions, de mousses ou encore sous forme de compositions pour aérosol comprenant également un agent propulseur sous pression.

Pour l'injection, la composition peut se présenter sous forme de lotion aqueuse, huileuse ou sous forme de sérum. Pour les yeux, elle peut se présenter sous forme de gouttes et pour l'ingestion, elle peut se présenter sous forme de capsules, de granulés, de sirops ou de comprimés.

Les quantités des différents constituants des compositions selon l'invention sont celles classiquement utilisées dans les domaines considérés.

Pour l'application topique sur la peau, ces compositions constituent notamment des crèmes de nettoyage, de protection, de traitement ou de soin pour le visage, pour les mains, pour les pieds, pour les grands plis anatomiques ou pour le corps, (par exemple crèmes de jour, crèmes de nuit, crèmes démaquillantes, crèmes de fond de teint, crèmes anti-solaires), des fonds de teint fluides, des laits de démaquillage, des laits corporels de protection ou de soin, des laits anti-solaires, des lotions, gels ou mousses pour le soin de la peau, comme des lotions de nettoyage, des lotions anti-solaires, des lotions de bronzage artificiel, des compositions pour le bain, des compositions désodorisantes comprenant un agent bactéricide, des gels ou lotions après-rasage, des crèmes épilatoires, des compositions contre les piqûres d'insectes, des compositions anti-douleur, des compositions pour traiter certaines maladies de la peau comme l'eczéma, la rosasée, le psoriasis, les lichens, les prurits sévères, les dermatites.

Les compositions peuvent aussi être conditionnées sous forme de composition pour aérosol comprenant également un agent propulseur sous pression.

La fraction ribosomale d'au moins une bactérie filamenteuse non photosynthétique utilisée selon l'invention peut aussi être incorporée dans diverses compositions pour soins capillaires, et notamment des shampooings, des lotions de mise en plis, des lotions traitantes, des crèmes ou des gels coiffants, des compositions de teintures (notamment teintures d'oxydation) éventuellement sous forme de shampooings colorants, des lotions restructurantes pour les cheveux, des compositions de permanente (notamment des compositions pour le premier temps d'une permanente), des lotions ou des gels antichute, des shampooings antiparasitaires, etc.

Les compositions peuvent aussi être à usage bucco-dentaire, par exemple une pâte dentifrice. Dans ce cas, les compositions peuvent contenir des adjuvants et additifs usuels pour les compositions à usage buccal et notamment des agents tensioactifs, des agents épaississants, des agents humectants, des agents de polissage tels que la silice, divers ingrédients actifs comme les fluorures, en particulier le fluorure de sodium, et éventuellement des agents édulcorants comme le saccharinate de sodium.

Lorsque la composition est une émulsion, la proportion de la phase grasse peut aller de 5 % à 80 % en poids, et de préférence de 5 % à 50 % en poids par rapport au poids total de la composition. Les huiles, les cires, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition est une solution ou un gel huileux, la phase grasse peut représenter plus de 90 % du poids total de la composition.

De façon connue, toute composition cosmétique peut contenir également des adjuvants habituels dans le domaine cosmétique, tels que les gélifiants hydrophiles ou lipophiles, les additifs hydrophiles ou lipophiles, les conservateurs, les antioxydants, les solvants, les parfums, les charges, les filtres, les absorbeurs d'odeur et les matières colorantes. Les quantités de ces différents adjuvants sont celles classiquement utilisées dans le domaine cosmétique, et par exemple de 0,01 % à 10 % du poids total de la composition. Ces adjuvants, selon leur nature, peuvent être introduits dans la phase grasse, dans la phase aqueuse et/ou dans les sphérules lipidiques.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

La composition peut contenir d'autres actifs hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.

Comme actifs lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Selon l'invention on peut, entre autres, associer une fraction ribosomale d'au moins une bactérie filamenteuse non photosynthétique à d'autres agents actifs destinés notamment à la prévention et/ou au traitement des affections de la peau. Parmi ces agents actifs, on peut citer à titre d'exemple :
- les agents modulant la différenciation et/ou la prolifération et/ou la pigmentation de la peau tels que l'acide rétinoïque et ses isomères, le rétinol et ses. esters, la vitamine D et ses dérivés, les oestrogènes tels que l'oestradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents antiviraux tels que l'acyclovir ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoïque ou le peroxyde de benzoyle.

Ainsi, selon un mode particulier, l'invention concerne une composition contenant au moins un fraction ribosomale d'au moins une bactérie filamenteuse non photosynthétique et au moins un agent choisi parmi les agents antibactériens, antiparasitaires, antifongiques, antiviraux, anti-inflammatoires, antiprurigineux, anesthésiques, kératolytiques, anti-radicaux libres, anti-séborrhéiques, antipelliculaires, antiacnéiques et/ou les agents modulant la différenciation et/ou la prolifération et/ou la pigmentation cutanée.

La présente invention a en outre pour objet un procédé de traitement cosmétique en vue de stimuler le système immunitaire et en particulier le système immunitaire de la peau, caractérisé par le fait que l'on applique sur la peau, sur les cheveux, et/ou sur les muqueuses, une composition telle que décrite ci-dessus.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs, application d'une lotion pour cheveux sur cheveux mouillés, de shampooings, ou encore application de dentifrice sur les gencives.

Les exemples et compositions suivants illustrent l'invention sans la limiter aucunement. Dans les compositions les proportions indiquées sont des pourcentages en poids, sauf mention contraire.

### Exemple 1 :

### Préparation d'une fraction ribosomale de Vitreoscilla filiformis :

Une culture de *Vitreoscilla filiformis* est effectuée selon le procédé décrit par la demanderesse dans la demande de brevet français publiée sous le numéro 2700172.

100 ml de culture sont alors centrifugés 10 minutes à 13000 tours par minute à 4°C.
Le surnageant est récupéré. On ajoute du sulfate d'ammonium à pH 7.5 à raison de 210 mg/ml.
Une nouvelle centrifugation est opérée dans les mêmes conditions que précédemment.
Le surnageant est dialysé (membranes de dialyse spectra, porosité 1000 MWCO (environ 1000 Daltons) contre un tampon Tris-HCl pH 7.6.

On obtient 70 ml de milieu enrichi en ribosomes.

### Exemple 2 :

### Effet d'une fraction ribosomale de Vitreoscilla filiformis sur la croissance et sur la différenciation des splénocytes de souris Balb/c de 7 semaines :

La fraction ribosomale de *Vitreoscilla filiformis* de l'exemple 1 est testée à différentes concentrations dans un modèle représentatif de l'activité d'un produit sur le système immunitaire : croissance et différenciation des splénocytes de souris Balb/c agées de 7 semaines.

L'activité potentielle de la fraction ribosomale est évaluée :
a) par la mesure de l'incorporation de la thymidine dans l'acide désoxyribonucléique (indice de prolifération) ;

Le témoin négatif est représenté par l'effet du milieu de culture sur les splénocytes et le témoin positif par l'effet de lipopolysaccharides (LPS) de référence à 0,001% et 0,0001%. Les LPS utilisés sont des LPS d*'Escherichia coli,* vendus par la société Sigma.

Procédure d'incorporation de thymidine tritiée :
Les splénocytes de souris sont répartis dans des boites de culture NUNC 96 puits, fond rond à raison de 250.000 cellules par puits dans un volume final de 200 ml. Les produits à tester sont ajoutés dans les puits à différentes concentrations. La prolifération cellulaire est évaluée par incorporation de thymidine tritiée (1 µCi/puits). 18 heures avant la fin de la culture 10 µl de thymidine tritiée à 100 µCi/ml sont ajoutés dans chaque puits de culture. 18 heures plus tard, les cellules sont récupérées sur un filtre à l'aide d'un récolteur de cellules type Tomtec. Le filtre est ensuite séché et recouvert d'un liquide de scintillation. La radioactivité déposée sur le filtre est déterminée par comptage dans un compteur de type β.
La prolifération est calculée par un indice de stimulation selon la formule suivante :

### Radioactivité test - Radioactivité contrôle (cellules + milieu) Radioactivité contrôle

Les résultats sont présentés dans le tableau ci-après :
(valeur des indices de stimulation calculés selon la formule ci-dessus)

| | 24 H | 48 H | 72 H | 96 H |
|---|---|---|---|---|
| Fraction ribosomale | | | | |
| 5 % | 1,02 | 4,08 | 3,10 | 1,97 |
| 2,5 % | 1,84 | 8,18 | 8,03 | 2,87 |
| 1,25 % | 1,64 | 4,80 | 2,03 | 0,89 |
| 0,63 % | 1,61 | 4,56 | 3,81 | 1,33 |
| 0,31 % | 1,63 | 3,97 | 4,66 | 4,16 |
| LPS | | | | |
| 0,001 % | 1,50 | 5,47 | 13,64 | 16,48 |
| 0,0001 % | 1,03 | 1,03 | 2,47 | 5,32 |

La fraction ribosomale augmente très significativement les indices de prolifération . L'effet maximal est obtenu à la concentration de 2.5 % et après 48 heures de culture.
L'effet est comparable à celui obtenu avec le témoin positif.
b) par la mesure de la production d'immunoglobulines après différenciation des splénocytes totaux en cellules B (lymphocytes B).

Le témoin négatif est représenté par l'effet du milieu de culture sur les splénocytes et le témoin positif par l'effet de lipopolysaccharides (LPS) de référence à 0,001% et 0,0001%. Les LPS utilisés sont des LPS d'*Escherichia coli,* vendus par la société Sigma.

Dosage des immunoglobulines :

Les cellules sont mises en culture dans les mêmes conditions que celles décrites précédemment. Après 2 jours de culture, les surnageants sont prélevés et dosés pour la présence d'immunoglobulines (lg).

Ce dosage est réalisé à l'aide d'un Kit Pharmingen, selon le protocole du fournisseur.

Les immunoglobulines sont des protéines présentes dans le sang qui se combinent aux antigènes afin que ces derniers soient reconnus et identifiés comme corps étranger par les lymphocytes B.

Les immunoglobulines sont subdivisées en différents types et sous-types en fonction de la structure de la partie constante de leur chaîne lourde. Chez la souris, on distingue 5 classes (lg M, G, A, D et E). L'immunoglobuline G est l' immunoglobuline la plus abondante dans les fluides corporels notamment extravasculaires où elle combat microorganismes et toxines. On peut classer les immunoglobulines G en 4 sous-classes (1, 2, 3 et 4).

L'immunoglobuline A est l'immunoglobuline majeure dans les sécrétions séro-muqueuses où elle défend les surfaces externes du corps.

L'immunoglobuline M est un agent agglutinant très efficace produit très tôt dans la réponse immunitaire. Elle constitue la première ligne de défense contre les bactériémies.

Seules les lg G1, G2a, G2b, G3, M et A ont été dosées dans ce test.

Les résultats sont présentés dans le tableau ci-après

| | IgG 1 | IgG 2a | IgG 2b | IgG 3 | IgM | IgA |
|---|---|---|---|---|---|---|
| Témoin | 1 | 1 | 1 | 1 | 1 | 1 |
| LPS 0,001 | 3,4 | 3,7 | 3,2 | 3,4 | 2,9 | 3,5 |
| LPS 0,0001 | 3 | 3,3 | 3,1 | 3,2 | 3 | 3 |
| F.R.5 % | 2,1 | 2,2 | 2,2 | 2,1 | 2,2 | 2,3 |
| F.R. 2,5 % | 3,3 | 3,3 | 3 | 3,1 | 2,9 | 3,4 |
| F.R. 1,25 % | 3,8 | 3,9 | 3,4 | 3,6 | 3,3 | 4 |

| | | | | | | |
|---|---|---|---|---|---|---|
| F.R. = fraction ribosomale. | | | | | | |

La fraction ribosomale augmente significativement la production d'immunoglobulines de toutes les classes et sous-classes étudiées. La concentration active optimale est de 1.25%.

Ces résultats sont comparables à ceux obtenus avec le témoin positif.

### Exemple 3 :

Exemple de compositions selon l'invention. Ces compositions ont été préparées par les méthodes classiques connues de l'homme du métier.

| Crème de nettoyage | | |
|---|---|---|
| Fraction ribosomale de l'exemple 1 | | 0.5 |
| Alcool Cétylique | | 2.00 |
| Stéarate de Glycérol | | 2.00 |
| Acide Stéarique | | 2.00 |
| Polyglyceryl-3 Hydroxylauryl Ether | | 5.00 |
| Huile Minérale Codex | | 12.00 |
| Carbomer | | 0.35 |
| Hydroxyde de Sodium | | 0.15 |
| Parfum | qsp | |
| Méthyl Paraben | | 0.20 |
| Eau déminéralisée Stérile | qsp | 100.00 |

| Lait de nettoyage | | |
|---|---|---|
| Fraction ribosomale de l'exemple 1 | | 0.5 |
| Carbomer | | 0.40 |
| Hydroxyde de Sodium | | 0.10 |
| Huile Minérale Codex | | 5.00 |
| Stéarate de Glycérol | | 1.00 |
| Alcool Cétylique | | 0.50 |
| PEG 100 Stéarate | | 0.80 |
| Méthyl Paraben | | 0.20 |
| Parfum | qsp | |
| Eau déminéralisée Stérile | qsp | 100.00 |

| Lotion de soin | | |
|---|---|---|
| Fraction ribosomale de l'exemple 1 | | 1 |
| Glycérol | | 2.00 |
| Méthyl Paraben | | 0.15 |
| Parfum | qsp | |
| Eau déminéralisée Stérile | qsp | 100.00 |

| Crème de soin | | |
|---|---|---|
| Fraction ribosomale de l'exemple 1 | | 2 |
| Stéarate de Glycérol | | 1.00 |
| PEG 100 Stéarate | | 1.00 |
| Acide Stéarique | | 1.00 |
| Alcool Cétylique | | 2.00 |
| Huile de Soja | | 3.00 |
| Huile de-Palme | | 2.00 |
| Cyclométhicone | | 2.00 |
| Diméthicone | | 1.00 |
| Polyacrylamide | | 0.20 |
| Glycérol | | 3.00 |
| Méthyl Paraben | | 0.20 |
| Parfum | qsp | |
| Eau Stérile Déminéralisée | qsp | 100.00 |

## Revendications

1. Fraction ribosomale préparée à partir d'au moins une bactérie filamenteuse non photosynthétique.

2. Fraction ribosomale selon la revendication 1, caractérisée en ce que ladite bactérie appartient à l'ordre des Beggiatoales.

3. Fraction ribosomale selon l'une quelconque des revendications précédentes, caractérisée en ce que ladite bactérie appartient au genre *Beggiatoa, Vitreoscilla, Flexithrix* ou *Leucothrix.*

4. Fraction ribosomale selon l'une quelconque des revendications précédentes, caractérisée en ce que ladite bactérie est choisie parmi des souches de *Vitreoscilla filiformis.*

5. Composition cosmétique ou pharmaceutique, caractérisée en ce qu'elle comprend au moins une fraction ribosomale telle que définie selon l'une quelconque des revendications 1 à 4.

6. Composition selon la revendication 5, caractérisée en ce que la fraction ribosomale est un agent immunostimulant.

7. Composition selon la revendication 6, caractérisée en ce que la fraction ribosomale est un agent immunostimulant du système immunitaire de la peau.

8. Composition selon l'une quelconque des revendications 5 à 7, caractérisée en ce que la fraction ribosomale représente de 0,0001 % à 20 % du poids total de la composition.

9. Composition selon la revendication 8, caractérisée en ce que la fraction ribosomale représente de 0,01 % à 10 % du poids total de la composition.

10. Procédé de traitement cosmétique, caractérisé en ce que l'on applique sur la peau, sur les cheveux et/ou sur les muqueuses au moins une composition cosmétique telle que définie selon l'une quelconque des revendications 5 à 9.

## Claims

1. Ribosomal fraction prepared from at least one nonphotosynthetic filamentous bacterium.

2. Ribosomal fraction according to Claim 1, characterized in that the said bacterium belongs to the order of the Beggiatoales.

3. Ribosomal fraction according to either of the preceding claims, characterized in that the said bacterium belongs to the genus *Beggiatoa, Vitreoscilla, Flexithrix* or *Leucothrix.*

4. Ribosomal fraction according to any one of the preceding claims, characterized in that the said bacterium is chosen from strains of *Vitreoscilla filiformis.*

5. Cosmetic or pharmaceutical composition characterized in that it includes at least one ribosomal fraction as defined according to any one of Claims 1 to 4.

6. Composition according to Claim 5, characterized in that the ribosomal fraction is an immunostimulant agent.

7. Composition according to Claim 6, characterized in that the ribosomal fraction is an immunostimulant agent for the immune system of the skin.

8. Composition according to any one of Claims 5 to 7, characterized in that the ribosomal fraction represents from 0.0001% to 20% of the total weight of the composition.

9. Composition according to Claim 8, characterized in that the ribosomal fraction represents from 0.01% to 10% of the total weight of the composition.

10. Process of cosmetic treatment, characterized in that at least one cosmetic composition as defined according to any one of Claims 5 to 9 is applied to the skin, to the hair and/or to the mucosae.

## Patentansprüche

1. Ribosomenfraktion, die aus mindestens einem filamentebildenden, nicht photosynthetischen Bakterium hergestellt ist.

2. Ribosomenfraktion nach Anspruch 1, dadurch gekennzeichnet, daß das Bakterium aus der Ordnung Beggiatoales stammt.

3. Ribosomenfraktion nach einem der vorhergehenden Ansprüche, dadurch gekennzeichnet, daß das Bakterium aus der Gattung *Beggiatoa, Vitreoscilla, Flexithrix* oder *Leucothrix* stammt.

4. Ribosomenfraktion nach einem der vorhergehenden Ansprüche, dadurch gekennzeichnet, daß das Bakterium unter den Stämmen von *Vitreoscilla filiformis* ausgewählt ist.

5. Kosmetische oder pharmazeutische Zusammensetzung, dadurch gekennzeichnet, daß sie mindestens eine Ribosomenfraktion nach einem der Ansprüche 1 bis 4 enthält.

6. Zusammensetzung nach Anspruch 5, dadurch gekennzeichnet, daß die Ribosomenfraktion ein Immunstimulanz ist.

7. Zusammensetzung nach Anspruch 6, dadurch gekennzeichnet, daß die Ribosomenfraktion ein Immunstimulanz für das Immunsystem der Haut ist.

8. Zusammensetzung nach einem der Ansprüche 5 bis 7, dadurch gekennzeichnet, daß die Ribosomenfraktion 0,0001 bis 20 % des Gesamtgewichts der Zusammensetzung ausmacht.

9. Zusammensetzung nach Anspruch 8, dadurch gekennzeichnet, daß die Ribosomenfraktion 0,01 bis 10 % des Gesamtgewichts der Zusammensetzung ausmacht.

10. Verfahren zur kosmetischen Behandlung, dadurch gekennzeichnet, daß auf die Haut, die Haare und/oder die Schleimhäute mindestens eine Zusammensetzung nach einem der Ansprüche 5 bis 9 aufgebracht wird.
